# FASCICULE DE BREVET EUROPEEN

(11) **EP 3 770 150 B1**
(45) Date de publication et mention de la délivrance du brevet: **25.01.2023**
(21) Numéro de dépôt: 20186794.2
(22) Date de dépôt: 20.07.2020
(51) Int. Cl.: C07D 311/04, C07D 493/04

(54) **PROCÉDÉ DE FABRICATION DE CHROMENES PAR CATALYSE AUX SELS DE CUIVRE DESTINÉS À LA PRÉPARATION DE RESINES THERMODURCISSABLES**
VERFAHREN ZUR HERSTELLUNG VON CHROMENEN DURCH KUPFERSALZKATALYSE, DIE FÜR DIE HERSTELLUNG VON WÄRMEHÄRTBAREN HARZEN BESTIMMT SIND
METHOD FOR MANUFACTURING CHROMENES BY COPPER SALT CATALYSIS INTENDED FOR THE PREPARATION OF HEAT-SETTING RESINS

(30) Priorité: 23.07.2019 FR 1908323
(43) Date de publication de la demande: 27.01.2021
(73) Titulaire: ArianeGroup SAS, 78130 Les Mureaux (FR); CENTRE NATIONAL DE LA RECHERCHE SCIENTIFIQUE (C.N.R.S.), 75016 Paris (FR); Université De Reims Champagne-Ardenne, 51100 Reims (FR)
(72) Inventeur: POUYET, Robin, 63800 COURNON (FR); COQUERET, Xavier, 51100 REIMS (FR); DEFOORT, Brigitte, 33160 Saint-Médard-en-Jalles (FR); RIVIERES, Bastien, 81640 LE SEGUR (FR)
(74) Mandataire: Cabinet Beau de Loménie

(56) Documents cités:
- EP-A2- 0 350 747
- WO-A1-2017/129661
- Michael S. Christodoulou et al.: "Divergent Palladium- and Platinum-Catalyzed Intramolecular Hydroamination/Hydroarylation of O-Propargyl-2-aminophenols", European Journal of Organic Chemistry, vol. 44 19 juillet 2018 (2018-07-19), pages 6176-6184, XP055693802, Wiley Online Library Extrait de l'Internet: URL:https://chemistry-europe.onlinelibrary .wiley.com/doi/full/10.1002/ejoc.201801103 [extrait le 2020-05-11]

## Description

La présente invention concerne le domaine des résines thermodurcissables, et des matériaux obtenus à partir de ces résines. Ces résines visent à remplacer les résines phénoliques dans toutes les applications dans lesquelles celles-ci sont utilisées, et notamment les matériaux dits « ablatifs ».

Un matériau ablatif est défini comme un matériau qui est capable de subir une ablation, c'est-à-dire une perte de substance par décomposition chimique, changement d'état ou érosion mécanique sous l'effet d'un flux de matière ou de rayonnement (Journal Officiel de la République Française du 22 septembre 2000). C'est en particulier le cas des matériaux qui entrent dans la constitution des boucliers thermiques destinés à l'aérospatiale et des parois de tuyères des moteurs à propulsions. Typiquement, dans ce cas, la couche externe du matériau ablatif qui est directement en contact avec l'environnement, subit sous l'effet de la chaleur une transformation chimique, ainsi qu'une récession liée à cette transformation. Cette couche externe rayonne donc vers l'extérieur et sa transformation chimique consomme de l'énergie. Ces deux effets contribuent à une moindre transmission de la chaleur vers les couches internes du matériau et donc à une isolation thermique de la structure sous-jacente. Un bon matériau ablatif doit être tel que sa transformation chimique sous l'effet de la chaleur soit endothermique, sa conductivité thermique soit faible en régime stationnaire et/ou en transitoire et sa transformation chimique ne s'accompagne pas d'une récession trop rapide. En particulier, pour remplir ce dernier point, il faut que la transformation chimique du matériau ablatif s'accompagne de la formation d'une croûte à base de carbone ou silice provenant de la pyrolyse de la résine.

Ceci est en particulier obtenu pour des résines ayant un taux de coke élevé. Le taux de coke est défini comme la masse de résidu qui est obtenu quand on décompose un échantillon d'un polymère organique par pyrolyse, à une température de 900°C sous atmosphère inerte (azote ou argon), rapportée à la masse initiale de cet échantillon. Les résines les plus intéressantes présentent un taux de coke supérieure à 50%.

Les résines phénoliques présentent en général un tel taux de coke et sont obtenues par polycondensation de monomères issus de la pétrochimie : le phénol et le formaldéhyde, ce qui leur vaut d'être aussi appelées résines phénol-formaldéhydes ou résines formophénoliques. Les précurseurs des résines phénoliques, le phénol et le formaldéhyde, sont respectivement CMR 2 et 1B. Ces deux composés sont donc sous la surveillance du Règlement (CE) n° 1907/2006 du Parlement Européen (REACH) qui vise à mieux protéger la santé humaine et l'environnement contre les risques liés aux substances chimiques. Il s'avère, de plus, que la polycondensation du phénol et du formaldéhyde n'est jamais achevée, d'où la présence de composés volatils et de molécules d'eau qui sont très difficiles à éliminer si un cycle thermique bien défini n'est pas suivi au cours de cette polycondensation et qui peuvent conduire à des matériaux poreux dans leur état natif ainsi qu'à des dégazages durant la vie des matériaux fabriqués à partir de résines phénoliques. Or, ces dégazages peuvent avoir des conséquences très néfastes dans certaines applications comme, par exemple, les applications aérospatiales.

Compte-tenu de la place qu'occupent actuellement les résines phénoliques dans l'industrie des matériaux plastiques et de ses inconvénients, de nouvelles résines thermodurcissables présentant des propriétés similaires à celles des résines phénoliques ont été obtenues à partir de précurseurs différents. Ainsi la demande de brevet WO2017/129661 décrit de telles résines et leurs procédés de fabrication. De telles résines ont un taux de coke supérieure à 50% et peuvent donc être utilisées comme matériaux ablatifs. Les précurseurs utilisés sont en particulier des molécules aromatiques porteuses de fonctions éther de propargyle. Toutefois l'énergie trop importante libérée pendant leur polymérisation pourrait engendrer un emballement thermique lors de la fabrication des matériaux composites. Ainsi, afin d'obtenir une enthalpie de polymérisation d'environ 800-900 J/g avec une perte de masse la plus faible possible lors de la polymérisation, il est nécessaire dans le procédé décrit dans cette demande de maintenir un traitement thermique long pendant la polymérisation afin de prévenir tout emballement thermique. Cette solution n'est donc pas optimisée vis-à-vis de la fabrication des pièces épaisses pouvant mesurer jusqu'à plusieurs dizaines de millimètres d'épaisseurs.

Les inventeurs se sont rendus compte qu'il était possible de diminuer l'énergie libérée pendant la polymérisation des résines à terminaisons éther de propargyle d'un facteur 6 par la conversion de la fonction éther de propargyle en fonction chromène et ainsi d'abaisser l'enthalpie de polymérisation jusqu'à une valeur < 500 J/g.

La demande de brevet EP0350747 décrit un procédé de préparation de résines thermodurcissables à partir de précurseurs chromènes, eux-mêmes obtenus par conversion d'éthers de propargyles aromatiques à l'aide d'une catalyse aux sels de cuivre ou de zinc. Toutefois, les solvants décrits explicitement dans cette demande page 5 lignes 27-28 qui sont le diéthylbenzène, le diisopropylbenzène, le dichlorobenzène, le trichlorobenzène et le tétralin ont un impact négatif sur l'environnement et/ou la santé humaine. En outre certains des éthers de propargyles aromatiques indiqués comme préférés dans cette demande tel que le bisphénol A propargylé ne permettent pas l'obtention de résines thermodurcissables ayant un taux de coke supérieure à 50%.

Un procédé de préparation de chromènes par conversion d'éthers de propargyles aromatiques utilisant comme catalyseur du iodure de cuivre dans le toluène est décrit dans le European Journal of Organic chemistry, 2018, volume 44, pages 6176-6184.

Les inventeurs ont découvert de façon surprenante que certains éthers de propargyles aromatiques étaient particulièrement intéressants pour la fabrication de résines thermodurcissables ayant un taux de coke supérieure à 50% et pour obtenir une enthalpie de polymérisation < 500 J/g lorsqu'ils étaient d'abord convertis en chromènes par catalyse homogène aux sels de cuivre, d'autant plus qu'ils sont issus de composés biosourçables tels que le résorcinol, l'eugénol, l'eugénol couplé et l'isoeugénol couplé. Ils ont en outre découvert qu'il était possible pour ces éthers de propargyles aromatiques particuliers d'utiliser l'anisole comme solvant au cours de cette catalyse, l'anisole étant un solvant meilleur pour la santé humaine tel que recommandé par l'initiative CHEM21 (consortium publique-privé visant le développement de méthodes durables pour l'industrie pharmaceutique). En particulier l'anisole peut être utilisé à une température inférieure à 185°C pour la conversion du résorcinol propargylé en chromène avec une catalyse homogène aux sels de cuivre ce qui évite la formation de précipité insoluble.

La présente invention concerne donc un procédé de fabrication de chromènes destinés à la préparation de résines thermodurcissables comprenant l'étape de transformation d'un éther de propargyle aromatique de formule générale (I) suivante : 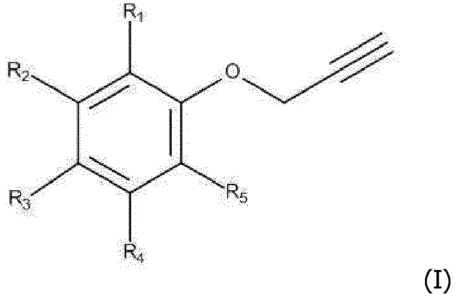 dans laquelle :
**R₁ et R₅** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₁ et R₅** représente un atome d'hydrogène ;
**R₂ et R₄** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle ;
**et R₃** représente un atome d'hydrogène, un groupe O-alkyle en C₁-C₆ ou
un groupe alcène en C₂-C₆, le groupe alcène étant éventuellement substitué par un groupe de formule générale (II) suivante : 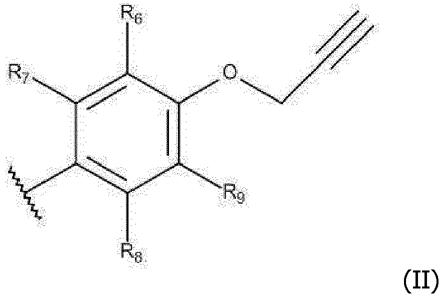 dans laquelle :
**R₆ et R₉** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₆ et R₉** représente un atome d'hydrogène ;
et **R₇ et R₈** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle;
à la condition que l'un au moins des **R₁, R₂, R₃, R₄ et R₅** ne représente pas un atome d'hydrogène ou un groupe O-alkyle en C₁-C₆ ;
et ses isomères cis/trans et ses isomères optiques et leurs mélanges racémiques
en un chromène par catalyse homogène aux sels de cuivre dans l'anisole à une température comprise entre 100 et 170°C, avantageusement entre 120 et 160°C, plus avantageusement entre 140 et 160°C, encore plus avantageusement entre 152 et 157°C, en particulier à 155°C.

Au sens de la présente invention on entend par « groupe alcène en C₂-C₆ » tout groupe alcène de 2 à 6 atomes de carbones, linéaire ou ramifié, en particulier le groupe vinyle, le groupe allyle ou le groupe but-2-ényle. Au sens de la présente invention on entend par « groupe alcyne en C₂-C₆ » tout groupe alcyne de 2 à 6 atomes de carbones, linéaire ou ramifié, en particulier le groupe éthynyle ou le groupe propargyle.

Au sens de la présente invention on entend par « groupe O-alkyle en C₁-C₆ » tout groupe O-alkyle de 1 à 6 atomes de carbones, linéaire ou ramifié, en particulier, le groupe méthoxy ou éthoxy.

Au sens de la présente invention on entend par « groupe O-alcène en C₂-C₆ » tout groupe O-alcène de 2 à 6 atomes de carbones, linéaire ou ramifié.

Au sens de la présente invention on entend par « groupe O-alcyne en C₂-C₆ » tout groupe O-alcyne de 2 à 6 atomes de carbones, linéaire ou ramifié en particulier le groupe O-propargyle.

Avantageusement l'éther de propargyle aromatique de formule générale (I) est choisi dans le groupe constitué par le résorcinol propargylé, l'eugénol propargylé, l'eugénol couplé propargylé, l'isoeugénol couplé propargylé, l'isoeugénol propargylé et leurs mélanges et leurs isomères cis/trans et leurs isomères optiques et leurs mélanges racémiques, plus avantageusement il s'agit du résorcinol propargylé. Ces produits sont bien connus de l'homme du métier et peuvent être préparés par des procédés bien connus, tels que ceux décrits dans la demande WO2017/129661. Ils ont l'avantage de pouvoir être issus de composés biosourçables tels que le résorcinol, l'eugénol, l'eugénol couplé, l'isoeugénol et l'isoeugénol couplé.

Le résorcinol propargylé a ainsi la formule générale suivante :

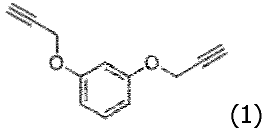

L'eugénol propargylé a ainsi la formule générale suivante :

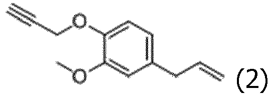

L'eugénol couplé propargylé a ainsi la formule générale suivante : 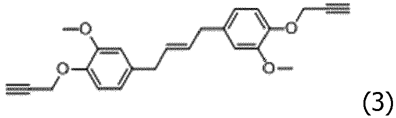 (isomère trans) ou la formule générale suivante : 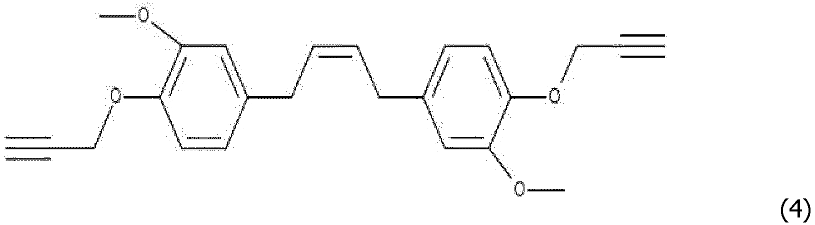 (isomère cis)
ou un mélange de ces deux isomères.

L'isoeugénol propargylé a ainsi la formule générale suivante :

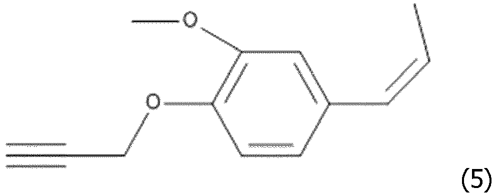

L'isoeugénol couplé propargylé a ainsi la formule générale suivante : 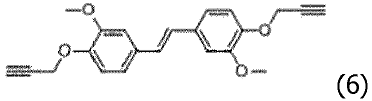 (isomère trans)
ou la formule générale suivante 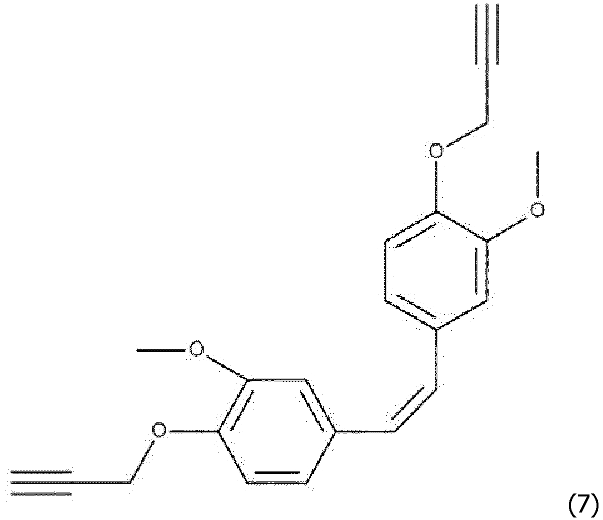 (isomère cis)
ou un mélange de ces deux isomères.

Le solvant utilisé dans le procédé selon l'invention est donc l'anisole. Avantageusement, le procédé selon la présente invention comprend une étape supplémentaire d'élimination de l'anisole, en particulier par évaporation, plus particulièrement après l'étape éventuelle d'élimination du catalyseur.

Le catalyseur du procédé selon la présente invention est un sel de cuivre. N'importe quel sel de cuivre (I) ou de cuivre (II) peut convenir, mais avantageusement le sel est choisi parmi les halogénures de cuivre, les acétates de cuivre, les bromures de cuivres, les chlorures de cuivre, les cyanures de cuivre, les fluorures de cuivre, les nitrates de cuivre, les phosphates de cuivre, les sulfates de cuivre, les alcoxydes de cuivre, les trifluoroacétates de cuivre, les méthanes sulfonates de cuivre, les benzènesulfonates de cuivre, les p-toluène sulfonates de cuivre, les tétrafluoroborates de cuivre, les hexafluoroborates de cuivre, les acétylacétonates de cuivre et les gluconates de cuivre. De façon avantageuse, le sel de cuivre est un chlorure de cuivre, en particulier choisi parmi le CuCI et le CuCl₂, avantageusement il s'agit du CuCI. Avantageusement, la teneur en catalyseur dans le milieu réactionnel est comprise entre 10 et 10 000 ppm, plus avantageusement entre 500 et 1500 ppm, encore plus avantageusement entre 300 et 1500 ppm, en particulier il est de 1000 ppm.

Le catalyseur peut être éliminé à la fin du procédé ou laissé dans le milieu réactionnel. Le procédé peut donc comprendre une étape supplémentaire d'élimination du catalyseur du milieu réactionnel, avantageusement par des procédés bien connus de l'homme du métier.

Le procédé selon l'invention avantageusement a lieu sous atmosphère inerte (azote ou argon par exemple).

La durée de réaction dépend des conditions réactionnelles telles que les éthers de propargyles aromatiques utilisées, la température de réaction, la teneur en catalyseur. Avantageusement le procédé selon l'invention dure entre 1 et 1000 heures, plus avantageusement entre 2 et 50 heures, encore plus avantageusement entre 3 et 48 heures.

Les inventeurs se sont aperçus qu'il n'était pas nécessaire d'avoir une conversion quantitative des fonctions éthers de propargyle en chromène pour obtenir une enthalpie de polymérisation inférieure à 500J/g. En effet l'énergie libérée pendant la polymérisation pour un substrat donné est dépendant de sa masse molaire et de sa fonctionnalité. En utilisant l'énergie libérée par fonction propargyle et par fonction chromène, combiné à la masse molaire de chaque substrat, il est possible de déterminer théoriquement le pourcentage de fonctions propargyle résiduelles maximum afin de se trouver en dessous des 500 J/g. ces valeurs ont été comparées aux valeurs expérimentales obtenues et sont similaires. Ainsi le tableau 1 ci-après indique le pourcentage de fonctions propargyle résiduelles théorique afin de se trouver en dessous des 500 J/g d'enthalpie de réaction lors de la polymérisation. Le pourcentage de fonctions propargyle résiduelles théorique est calculé de la façon suivante : (nombre de moles de fonctions propargyles à l'issue de la réaction) / (nombre de moles de fonctions propargyle avant le début de la réaction) x100.

**Tableau 1**

| **Substrat** | **M (g/mol)** | **Fonctionnalité en groupements propargyle** | **Pourcentage molaire de fonctions propargyles résiduelles théorique pour atteindre une enthalpie de 500 J/g** |
|---|---|---|---|
| Résorcinol propargylé | 186,21 | 2 | 11 |
| Eugénol couplé propargylé | 376,44 | 2 | 39 |
| Isoeugénol couplé propargylé | 348,39 | 2 | 35 |

Ainsi avantageusement, la conversion des éthers de propargyles aromatiques en chromène par le procédé selon la présente invention n'est pas totale et le chromène obtenu comprend des fonctions propargyles résiduelles. De façon avantageuse, le pourcentage molaire de fonctions propargyles résiduelles du chromène est inférieur à 11% lorsque l'éther de propargyle aromatique de formule générale (I) est le résorcinol propargylé, le pourcentage molaire de fonctions propargyles résiduelles du chromène est inférieur à 39% lorsque l'éther de propargyle aromatique de formule générale (I) est l'eugénol couplé propargylé et le pourcentage molaire de fonctions propargyles résiduelles du chromène est inférieur à 35% lorsque l'éther de propargyle aromatique de formule générale (I) est l'isoeugénol couplé propargylé.

Dans le cas où l'éther de propargyle aromatique de formule générale (I) est le résorcinol propargylé, le chromène obtenu à l'aide du procédé selon l'invention peut avoir la formule C et / ou D suivante, avantageusement il s'agit d'un mélange des formules C et D. 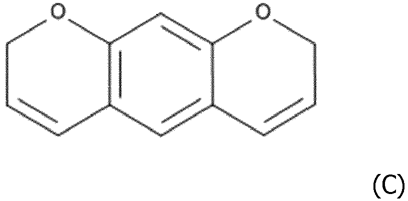 et

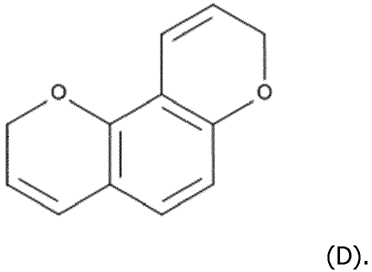

En particulier la proportion molaire de chromène de formule D dans le mélange après conversion totale est comprise entre 50 et 55% et celle de chromène de formule C entre 45 et 50%, en particulier elle est de 45% de chromène de formule C et 55% de chromène de formule D.

Dans le cas où l'éther de propargyle aromatique de formule générale (I) est le résorcinol propargylé, le chromène obtenu à l'aide du procédé selon l'invention peut également avoir la formule A et / ou B suivante.

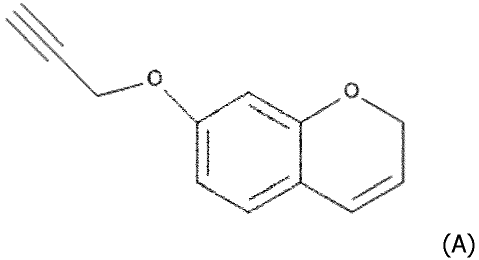

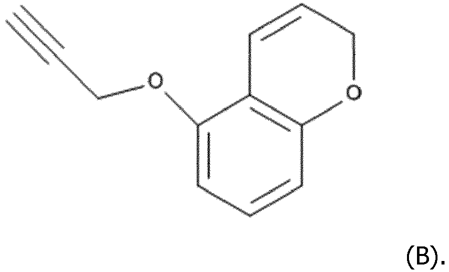

Toutefois ces molécules sont en général rapidement converties en composés de formule C et D.

Avantageusement, le rendement molaire de la réaction de conversion de l'éther de propargyle aromatique en chromène est compris entre 85 et 99%, en particulier entre 90 et 99%.

La présente invention concerne en outre un procédé de préparation d'un matériau en résine thermodurcie comprenant les étapes successives suivantes :
- a) mise en oeuvre du procédé selon la présente invention, avantageusement tel que décrit ci-dessus ;
- b) polymérisation du produit de la réaction obtenu à l'étape a) de façon à obtenir le matériau en résine thermodurcie.
- c) récupération du matériau en résine thermodurcie obtenu à l'étape b). Conformément à l'invention, la polymérisation de la résine peut être réalisée par tout moyen susceptible d'induire la polymérisation/réticulation du chromène et, notamment, par application d'un traitement thermique ou d'un traitement lumineux (lumière visible, UV ou IR).

En particulier l'étape b) est mise en oeuvre par traitement thermique, avantageusement à une température comprise entre 80°C et 180°C, plus avantageusement à l'aide de plusieurs paliers de chauffe (en particulier 5), sans ajout d'autres composants, tel que par exemple 2h à 80°C, 2h à 100°C, 2h à 120°C, 2h à 140°C et 2h à 180°C ou 1h à 80°C, 1h à 100°C, 1h à 120°C, 1h à 140°C, 1h à 160°C, 1h à 180°C, 1h à 200°C.

Plus particulièrement le procédé selon l'invention peut comprendre entre les étapes b) et c) une étape b1) de recuit à une température supérieure à 200°C mais inférieure à la température de dégradation de la résine, par exemple à 220°C. Cette étape permet d'améliorer les propriétés thermomécaniques de la résine.

De façon avantageuse, l'enthalpie de polymérisation de l'étape b) est inférieure à 500 J / g.

Avantageusement, le taux de coke de la résine thermodurcie obtenue à l'étape b) est supérieur à 50%.

Dans un mode de réalisation avantageux, le matériau en résine thermodurcie, est un matériau formant la matrice d'un matériau composite du type comprenant une matrice dans laquelle se trouve un renfort.

Le renfort présent dans le matériau composite peut être de différents types. Ainsi, il peut notamment s'agir d'un renfort constitué de fibres telles que des fibres de verre, des fibres de quartz, des fibres de carbone, des fibres de graphite, des fibres de silice, des fibres métalliques comme des fibres d'acier ou des fibres d'aluminium, des fibres de bore, des fibres céramiques comme des fibres de carbure de silicium ou de carbure de bore, des fibres organiques de synthèse comme des fibres d'aramide, des fibres de polyéthylène, des fibres de polyester ou des fibres de poly(p-phénylène benzobisoxazole), plus connues sous le sigle PBO, des fibres organiques naturelles comme des fibres de chanvre, des fibres de lin ou des fibres de soie, ou encore de mélanges de telles fibres, auquel cas ce renfort peut se présenter, selon la nature des fibres qui le constituent, sous la forme de fils coupés, de fibres broyées, de mats à filaments continus, de mats à filaments coupés, de stratifils (ou « rovings » en langue anglaise), de tissus, de tricots, de feutres, etc, ou encore sous la forme de complexes réalisés par association de différents types de matériaux plans.

II peut également s'agir d'un renfort constitué de particules telles que des particules de liège ou des charges réfractaires du type tungstène, oxyde de magnésium, oxyde de calcium, alumine, silice, dioxyde de zirconium, dioxyde de titane, ou oxyde de béryllium.

Par ailleurs, la fabrication du matériau composite, et donc l'ajout de renfort dans la résine, peut être réalisée par toutes les techniques connues de l'homme du métier des matériaux composites comme, par exemple, par imprégnation, par moulage par injection simultanée, par moulage par drapage autoclavé, par moulage sous vide, par moulage par injection basse pression de résine (ou RTM pour « Resin Transfert Molding »), par moulage à la presse à froid "voie humide" basse pression, par moulage par injection de compound (ou BMC pour « Bulk Molding Compound »), par moulage par compression de mats préimprégnés (ou SMC pour « Sheet Molding Compound »), par enroulement filamentaire, par centrifugation ou encore par pultrusion, l'imprégnation étant préférée dans le cas où le renfort est constitué de fibres.

De préférence, le matériau composite est un matériau composite ablatif et, plus spécifiquement, un matériau composite ablatif de protection thermique, notamment pour l'aérospatiale.

La présente invention sera mieux comprise à la lecture de la description des exemples qui suivent qui sont donnés à titre indicatifs.

### Exemple 1 : Conversion du résorcinol propargylé et préparation de la résine selon l'invention

### Synthèse du résorcinol propargylé

10g (0,091 mol) de résorcinol (Alfa Aesar) est solubilisé dans 50 mL de diméthylsulfoxyde (DMSO), 50g (0,363 mol) de carbonate de potassium (K₂CO₃) est broyé puis ajouté sous agitation magnétique et le milieu est chauffé à 70°C (ext). 14,45 mL (2,2 eq.) de chlorure de propargyle (ABCR) est ajouté au goutte à goutte. La réaction est contrôlée par CCM avec un éluant 7:3 éther de pétrole : diéthyl éther (volume). Après filtration et dilution dans 100 mL d'acétate d'éthyle le milieu est extrait avec 3x100 mL de saumure. La phase organique est séchée sur MgSO₄, filtrée et concentrée sous pression réduite. Le composé est purifié par distillation sous vide (T°C = 120°C, 4,5 Pa). Le rendement est de 77,4%.

### Conversion du résorcinol propargylé

10 grammes (0,054 mol) de résorcinol propargylé obtenu précédemment, 1000 ppm de CuCI et 100 mL d'anisole sont introduits dans un tube de schlenk surmonté d'un réfrigérant. Un bullage d'argon est effectué pendant 10 minutes et le milieu est conservé sous argon. Le tube de schlenk est placé dans un bain d'huile préchauffé à 155°C et la réaction est poursuivie à reflux pendant 4 heures. Après 4 heures, le milieu est filtré sur un filtre PTFE de 0,20 µm pour retirer les précipités éventuellement formés L'anisole est évaporée sous pression réduite à l'aide d'un four à boule et d'une pompe à palette. La proportion de fonction éther de propargyle résiduelle est estimée par RMN ¹H à 5%. Le rendement est de 99%. La proportion de fonctions éthers de propargyle résiduelles inférieure à 11% est conforme au cahier des charges fixé

### Polymérisation du chromène issu du résorcinol propargylé

La polymérisation des mélanges propargyle-chromène se fait par montée progressive en température. Dans le cas d'un mélange résorcinol propargyle-chromène avec une proportion en fonctions éthers de propargyle résiduelles inférieure à 11% tel qu'obtenu précédemment, le traitement thermique appliqué est le suivant : 2h à 80°C, 2h à 100°C, 2h à 110°C, 2h à 120°C, 2h à 130°C et 2h à 150°C.

Un recuit à 220°C peut être effectué pour augmenter les propriétés thermo-mécaniques.

Le taux de coke obtenu avant et après recuit est de 65,9% et l'enthalpie de réaction de 420 J/g.

### Exemple 2 : Conversion du résorcinol propargylé et préparation de la résine selon l'invention

### Conversion du résorcinol propargarlé

50 grammes (0,269 mol) de résorcinol propargylé obtenu selon le procédé indiqué dans l'exemple 1, 1000 ppm de CuCI et 150 mL d'anisole sont introduits dans un bicol de 500 mL équipé d'un agitateur magnétique et surmonté d'un réfrigérant. Un bullage d'argon est effectué pendant 10 minutes et le milieu est conservé sous argon. Le milieu est placé dans un bain d'huile préchauffé à 155°C et la réaction est poursuivie à reflux pendant 7 heures jusqu'à conversion totale des fonctions éthers de propargyle en fonctions chromène.. Après 7 heures, le milieu est filtré sur un filtre en cellulose pour retirer les précipités éventuellement formés L'anisole est évaporée par distillation sous pression réduite. La proportion de fonctions éthers de propargyle résiduelles est estimée par RMN ¹H inférieure à 1%. Le rendement est de 97%. La proportion de fonctions propargyle résiduelles inférieure à 11% est conforme au cahier des charges fixé.

### Polymérisation du chromène issu du résorcinol propargylé

Le procédé mis en oeuvre est identique à celui décrit dans l'exemple 1. Le taux de coke obtenu avant et après recuit est de 65,3% et l'enthalpie de réaction de 300 J/g.

### Exemple 3 : Conversion de l'eugénol propargylé et préparation de la résine selon l'invention

### Synthèse de l'eugénol propargarlé

L'eugénol (Sigma Aldrich) (200g), le K₂CO₃ (211g) et le diméthylformamide (DMF) (2000mL) sont introduits dans un ballon de 6L et sont chauffés à 75°C sous agitation mécanique. Le chlorure de propargyle (ABCR) à 70% dans le toluène (158,5 mL) est ajouté goutte à goutte à l'aide d'une ampoule de coulée et le milieu réactionnel est chauffé et agité à 75°C pendant la nuit. La réaction est contrôlée par CCM avec un éluant éther de pétrole/éther diéthylique 7:3 (volume). Après réaction, le milieu réactionnel est filtré puis dilué et rincé à l'acétate d'éthyle. La phase organique est rincée à l'eau jusqu'à décoloration de la phase aqueuse (4 fois). La phase organique est séchée sur MgSO₄ et concentrée sous vide. Le rendement du brut est de 93%. Le composé est purifié par distillation sous vide (p=4,5Pa et T°C = 60°C). Le rendement du composé distillé est de 90%.

### Conversion de l'eugénol propargylé

1g (0,005 mol) d'eugénol propargylé obtenu précédemment, 1000 ppm de CuCI et 3 mL d'anisole sont introduits dans un tube de schlenk. Un bullage d'argon est effectué pendant 10 minutes, et le milieu est conservé sous argon avec un réfrigérant. Le milieu est introduit dans un bain d'huile préchauffé à 155°C. La réaction est laissée à reflux et contrôlée par prélèvement régulier et analyse RMN ¹H. Après 23h, l'anisole est évaporée à l'évaporateur rotatif. La proportion de fonctions éthers de propargyle résiduelles est estimée par RMN ¹H inférieure à 1%. Le rendement est de 90%.

### Polymérisation du chromène issu de l'eugénol propargylé

Le procédé mis en oeuvre est identique à celui décrit dans l'exemple 1 pour le résorcinol propargylé.

### Exemple 4 : Conversion de l'eugénol couplé propargylé et préparation de la résine selon l'invention

### Synthèse de l'eugénol couplé

26,675g (0,1625 mol) d'eugénol (Sigma Aldrich) est introduit dans un ballon tricol de 50 mL avec un agitateur magnétique. 0,1337g (0,1%mol) de catalyseur de Grubbs (Sigma Aldrich) de 1ère génération est introduit à l'aide d'un pilulier à contrecourant d'argon. Le milieu est directement mis sous vide poussé (3 kPa) et laissé agiter pendant 12h. Un spectre RMN 1H du brut est effectué pour déterminer la conversion de l'eugénol. La conversion est de 67% molaire de composés eugénol couplé, les 37% molaire restant étant un mélange d'eugénol non réagit et de son isomère l'isoeugénol. La proportion stoechiométrique est évaluée par RMN 1H à 34,3%/65,7% pour les composés eugénol couplé cis- et trans-respectivement. Le milieu est solubilisé dans un minimum d'éther à reflux, puis laissé reposer à température ambiante. Le solide est filtré sous pression réduite sur un fritté porosité 4, puis lavé avec 4x20mL de cyclohexane. Le solide est séché sous vide poussé à l'aide d'une pompe à palette pendant 10h. Afin de retirer le catalyseur de Grubbs 1ère génération, le solide est solubilisé dans le dichlorométhane (DCM) puis filtré sur célite. Un dépôt noir est observé sur la célite. La phase organique est séchée sous pressions réduite. Le rendement obtenu est de 25%.

### Synthèse de l'eugénol couplé propargylé

3g (0,010 mol) d'eugénol couplé obtenu précédemment est solubilisé dans 30 mL (10eqm) de DMF. 5,52g (4eq.) Du carbonate de potassium finement broyé (K₂CO₃) est ajouté sous agitation magnétique. 2,78 mL (2,5eq.) de bromure de propargyle (Alfa Aesar) (80%m dans le toluène) est ajouté à l'aide de l'ampoule de coulée. L'agitation magnétique est maintenue pendant 12h. La complétion de la réaction est contrôlée par CCM avec un éluant éther de pétrole : Acétate d'éthyle 50:50 (volume). Après filtration du K₂CO₃ et lavage au DMF, un excès d'eau distillée (200 mL) est ajouté pour faire précipiter le produit. Le solide est récupéré. 200 mL d'acétate d'éthyle est ajouté dans le milieu pour l'extraction. La phase aqueuse est jetée. Le solide récupéré préalablement est redissout dans la phase organique. La phase organique est lavée 3 fois à l'eau distillée (3x100 mL) et 1 fois à la saumure (1×100mL). La phase organique est séchée sur MgSO₄, filtrée et concentrée sous pression réduite. Le rendement est de 90%.

### Conversion de l'eugénol couplé propargylé

0,5g (0,001 mol) d'eugénol couplé propargylé obtenu précédemment, 1000 ppm de CuCI et 10 mL d'anisole sont introduits dans un tube de schlenk. Un bullage d'argon est effectué pendant 10 minutes, et le milieu est conservé sous argon avec un réfrigérant. Introduire le tube de schlenk dans un bain d'huile préchauffé à 155°C. La réaction est contrôlée par prélèvement régulier et analyse RMN ¹H. Après 24h de réaction, l'anisole est évaporée au four à boule à l'aide d'une pompe à palette. La proportion de fonctions éthers de propargyle résiduelles est estimée par RMN ¹H à 9%. Le rendement est de 97%. La proportion de fonctions éthers de propargyle résiduelles inférieure à 39% est conforme au cahier des charges fixé.

### Polymérisation du chromène issu de l'eugénol couplé propargylé

Le procédé mis en oeuvre est identique à celui décrit dans l'exemple 1 pour le résorcinol propargylé à l'exception du fait que le traitement thermique appliqué est le suivant : 1h à 80°C, 1h à 100°C, 1h à 120°C, 1h à 140°C, 1h à 160°C, 1h à 180°C, 1h à 200°C.

### Exemple 5 : Conversion de l'isoeugénol couplé propargylé et préparation de la résine selon l'invention

### Synthèse de l'isoeugénol couplé

0,0181g (0,017%) de catalyseur de Grubbs II (UMICORE M2a) est introduit dans un ballon de 50 mL avec un agitateur magnétique, puis 20g (0,122 mol) d'isoeugénol (Sigma Aldrich) est ajouté. Le milieu est placé sous un flux d'argon et chauffé à 90°C. Le milieu devient solide après 3 minutes de réaction. Après refroidissement, un spectre RMN 1H du brut est effectué pour déterminer la conversion de l'isoeugénol en stilbène. Le taux de conversion est de 90%. Uniquement le composé trans- est observé. Le produit est récupéré par suspension dans 4vol. de DCM, le milieu est chauffé à reflux pendant 1heure jusqu'à complète solubilisation puis laissé au repos à température ambiante toute la nuit. La suspension est filtrée sur fritté et lavée avec 1 vol. de cyclohexane. Le rendement isolé est de 67%.

### Synthèse de l'isoeugénol couplé propargylé

10g (0,037 mol) d'eugénol stilbène obtenu précédemment est solubilisé dans 100mL (10eqm) de DMF. 25g (4,5eq.) Du carbonate de potassium finement broyé (K₂CO₃) est ajouté sous agitation magnétique. 10,23mL (2,5eq.) de bromure de propargyle (Alfa Aesar) (80%m dans le toluène) est ajouté à l'aide d'une seringue. L'agitation magnétique est maintenue pendant 12h. La complétion de la réaction est contrôlée par CCM avec un éluant éther de pétrole : Acétate d'éthyle 50:50 (volume). La conversion est totale après une nuit de réaction. Le K₂CO₃ est filtré puis lavé avec de l'acétate d'éthyle. Le composé est extrait avec 2x100 mL d'acétate d'éthyle. Les phases organiques sont lavées 4x100 mL à la saumure. Les phases organiques sont séchées sur MgSO₄, filtrées et concentrées sous pression réduite. Le rendement du produit est de 30%.

### Conversion de l'isoeugénol couplé propargylé

1g (0,003 mol) d'isoeugénol couplé propargylé obtenu précédemment, 1000 ppm de CuCI et 20 mL d'anisole sont introduits dans un tube de schlenk. Un bullage d'argon est effectué pendant 10 minutes, et le milieu est conservé sous argon avec un réfrigérant. Introduire le tube de schlenk dans un bain d'huile préchauffé à 155°C. La réaction est contrôlée par prélèvement régulier et analyse RMN ¹H. Après 48h de réaction, l'anisole est évaporée au four à boule à l'aide d'une pompe à palette. La viscosité de la résine n'a pas permis d'évaporer complètement le solvant de réaction. La proportion de fonctions éthers de propargyle résiduelles est estimée par RMN ¹H à 12%. La proportion de fonctions éthers de propargyle résiduelles inférieure à 39% est conforme au cahier des charges fixé.

### Polymérisation du chromène issu de l'isoeugénol couplé propargylé

Le procédé mis en oeuvre est identique à celui décrit dans l'exemple 1.

### Exemple 5 : Conversion de l'isoeugénol propargylé et préparation de la résine selon l'invention

### Synthèse de l'isoeugénol propargylé

20g (0,130 mol) d'isoeugénol (Sigma Aldrich) est solubilisé dans 100 mL (5eqm) de DMF. 33,67g (2eq.) de carbonate de potassium finement broyé (K₂CO₃) est ajouté sous agitation magnétique. 20,35 mL (1,5eq.) de bromure de propargyle (Alfa Aesar) (80%m dans le toluène) est ajouté à l'aide de l'ampoule de coulée. L'agitation magnétique est maintenue pendant 12h. La complétion de la réaction est contrôlée par CCM avec un éluant éther de pétrole : Acétate d'éthyle 70:30 (volume). Après filtration du K₂CO₃ et lavage à l'acétate d'éthyle. 100 mL d'acétate d'éthyle est ajouté dans le milieu pour l'extraction. La phase organique est lavée 3 fois à l'eau distillée (3x100 mL) et 1 fois à la saumure (1x100 mL). La phase organique est séchée sur MgSO₄, filtrée et concentrée sous pression réduite. Le rendement est de 91%.

Le composé est purifié par distillation sous vide au four à boule (p= 15 Pa et T°C chauffage = 140°C). Le composé est récupéré sous forme de cristaux blancs. Le rendement global après purification est de 73%.

### Conversion de l'isoeugénol propargylé

1g (0,005 mol) d'isoeugénol propargylé obtenu précédemment, 1000 ppm de CuCI et 5 mL d'anisole sont introduits dans un tube de schlenk surmonté d'un réfrigérant. Un bullage d'argon est effectué pendant 10 minutes et le milieu est conservé sous argon. Le tube de schlenk est introduit dans un bain d'huile préchauffé à 155°C et la réaction est laissée à reflux. L'avancement de la réaction est contrôlé par prélèvement régulier et analyse RMN ¹H. Après 30 heures de réaction, l'anisole est évaporée par distillation à 80°C à l'aide d'un four à boules. Le rendement est de 98%. La proportion de fonctions éthers de propargyle résiduelles est estimée par RMN ¹H à 10%.

### Polymérisation du chromène issu de l'isoeugénol propargylé

Le procédé mis en oeuvre est identique à celui décrit dans l'exemple 1.

## Revendications

1. Procédé de fabrication de chromènes destinés à la préparation de résines thermodurcissables comprenant l'étape de transformation d'un éther de propargyle aromatique de formule générale (I) suivante : 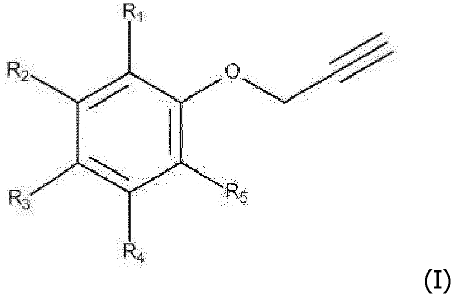 dans laquelle :
**R₁ et R₅** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆, à la condition que l'un au moins des **R₁ et R₅** représente un atome d'hydrogène ;
**R₂ et R₄** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle;
**et R₃** représente un atome d'hydrogène, un groupe O-alkyle en C₁-C₆ ou un groupe alcène en C₂-C₆, le groupe alcène étant éventuellement substitué par un groupe de formule générale (II) suivante 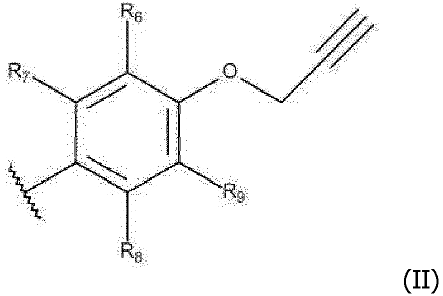 dans laquelle :
**R₆ et R₉** représentent indépendamment l'un de l'autre un atome d'hydrogène, un groupe alcène en C₂-C₆, alcyne en C₂-C₆, O-alkyle en C₁-C₆, O-alcène en C₂-C₆ ou O-alcyne en C₂-C₆,à la condition que l'un au moins des **R₆ et R₉** représente un atome d'hydrogène ;
et **R₇ et R₈** représentent indépendamment l'un de l'autre un atome d'hydrogène, un groupe alcène en C₂-C₆, alcyne en C₂-C₆ tel qu'un propargyle, O-alkyle en C₁-C₆, O-alcène en C₂-C₆ ou O-alcyne en C₂-C₆ tel qu'un O-propargyle ;
à la condition que l'un au moins des **R₁, R₂, R₃, R₄ et R₅** ne représente pas un atome d'hydrogène ou un groupe O-alkyle en C₁-C₆ ;
et ses isomères cis/trans et ses isomères optiques et leurs mélanges racémiques
en un chromène par catalyse homogène aux sels de cuivre dans l'anisole à une température comprise entre 100 et 170°C, avantageusement entre 120 et 160°C.

2. Procédé selon la revendication 1, **caractérisé en ce que** l'éther de propargyle aromatique de formule générale (I) est choisi dans le groupe constitué par le résorcinol propargylé, l'eugénol propargylé, l'eugénol couplé propargylé, l'isoeugénol couplé propargylé, l'isoeugénol propargylé et leurs mélanges et leurs isomères cis/trans et leurs isomères optiques et leurs mélanges racémiques, avantageusement il s'agit du résorcinol propargylé.

3. Procédé selon la revendication 2, **caractérisé en ce que** le pourcentage molaire de fonctions propargyles résiduelles du chromène est inférieur à 11% lorsque l'éther de propargyle aromatique de formule générale (I) est le résorcinol propargylé, le pourcentage molaire de fonctions propargyles résiduelles du chromène est inférieur à 39% lorsque l'éther de propargyle aromatique de formule générale (I) est l'eugénol couplé propargylé et le pourcentage molaire de fonctions propargyles résiduelles du chromène est inférieur à 35% lorsque l'éther de propargyle aromatique de formule générale (I) est l'isoeugénol couplé propargylé.

4. Procédé selon l'une quelconque des revendications 1 à 3, **caractérisé en ce qu'**il dure entre 1 et 1000 heures, avantageusement entre 2 et 50 heures.

5. Procédé selon l'une quelconque des revendications 1 à 4, **caractérisé en ce que** le catalyseur est choisi parmi le CuCI et le CuCl₂, avantageusement il s'agit du CuCI.

6. Procédé selon l'une quelconque des revendications 2 à 5, **caractérisé en ce que** l'éther de propargyle aromatique de formule générale (I) est le résorcinol propargylé et **en ce que** le chromène obtenu a la formule C et / ou D suivante, avantageusement est un mélange des formules C et D 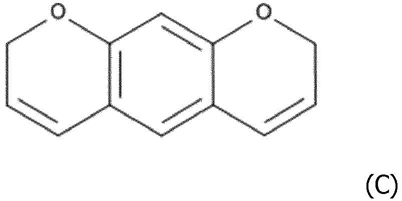 et

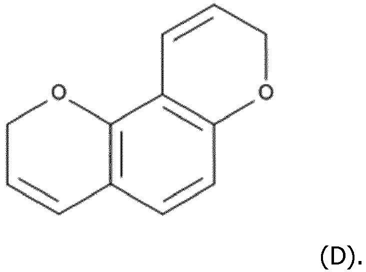

7. Procédé selon l'une quelconque des revendications 1 à 6, **caractérisé en ce que** la teneur en catalyseur dans le milieu réactionnel est comprise entre 10 et 10000ppm, avantageusement entre 500 et 1500 ppm.

8. Procédé de préparation d'un matériau en résine thermodurcie comprenant les étapes successives suivantes :
- a) mise en oeuvre du procédé selon l'une quelconque des revendications 1 à 7 ;
- b) polymérisation du produit de la réaction obtenu à l'étape a) de façon à obtenir le matériau en résine thermodurcie ;
- c) récupération du matériau en résine thermodurcie obtenu à l'étape b).

9. Procédé selon la revendication 8, **caractérisé en ce que** l'enthalpie de polymérisation de l'étape b) est inférieure à 500 J / g.

10. Procédé selon l'une quelconque des revendications 8 ou 9, **caractérisé en ce que** le taux de coke de la résine thermodurcie obtenue à l'étape c) est supérieur à 50%.

## Patentansprüche

1. Verfahren zur Herstellung von Chromenen, die für die Herstellung von durch Wärme aushärtbare Harze bestimmt sind, umfassend den Schritt der Umwandlung eines aromatischen Propargylethers der nachstehenden allgemeinen Formel (I): 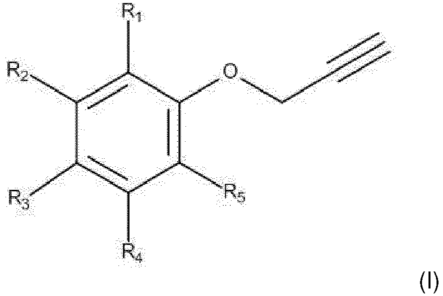 bei der:
R₁ und R₅ unabhängig voneinander ein Wasserstoffatom, eine C₂-C₆-Alken-, C₂-C₆-Alkin-, C₁-C₆ O-Alkyl-, C₂-C₆ O-Alken- oder C₂-C₆ O-Alkingruppe darstellen, mit der Maßgabe, dass wenigstens einer von R₁ und R₅ ein Wasserstoffatom darstellt,
R₂ und R₄ unabhängig voneinander ein Wasserstoffatom, eine C₂-C₆-Alken-, C₂-C₆-Alkin-, wie ein Propargyl, eine C₁-C₆ O-Alkyl-, C₂-C₆ O-Alken- oder C₂-C₆ O-Alkingruppe, wie ein O-Propargyl, darstellen,
und R₃ ein Wasserstoffatom, eine C₁-C₆ O-Alkylgruppe oder eine C₂-C₆-Alkengruppe darstellt, wobei die Alkengruppe eventuell durch eine Gruppe der nachstehenden allgemeinen Formel (II) substituiert ist 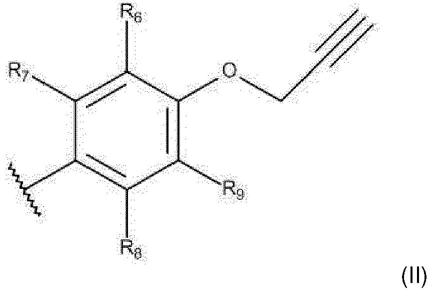 bei der:
R₆ und R₉ unabhängig voneinander ein Wasserstoffatom, eine C₂-C₆-Alken-, C₂-C₆-Alkin-, C₁-C₆ O-Alkyl-, C₂-C₆ O-Alken- oder C₂-C₆ O-Alkingruppe darstellen, mit der Maßgabe, dass wenigstens einer von R₆ und R₉ ein Wasserstoffatom darstellt,
und R₇ und R₈ unabhängig voneinander ein Wasserstoffatom, eine C₂-C₆-Alken-, C₂-C₆-Alkin-, wie ein Propargyl, eine C₁-C₆ O-Alkyl-, C₂-C₆ O-Alken- oder C₂-C₆ O-Alkingruppe, wie ein O-Propargyl, darstellen,
mit der Maßgabe, dass wenigstens einer von R₁, R₂, R₃, R₄ und R₅ kein Wasserstoffatom oder keine C₁-C₆ O-Alkylgruppe darstellt,
und seine cis/trans-Isomere und seine optischen Isomere und ihre racemischen Mischungen
in ein Chromen durch homogene Katalyse mit Kupfersalzen in Anisol bei einer Temperatur im Bereich zwischen 100 und 170 °C, vorteilhafterweise zwischen 120 und 160 °C.

2. Verfahren nach Anspruch 1, **dadurch gekennzeichnet, dass** der aromatische Propargylether der allgemeinen Formel (I) ausgewählt ist aus der Gruppe bestehend aus Propargylresorcin, Propargyleugenol, gekoppeltem Propargyleugenol, gekoppeltem Propargylisoeugenol, Propargylisoeugenol und deren Mischungen und deren cis /trans-Isomeren und ihren optischen Isomeren und ihren racemischen Mischungen, vorteilhafterweise handelt es sich um Propargylresorcin.

3. Verfahren nach Anspruch 2, **dadurch gekennzeichnet, dass** der prozentuale Molanteil an restlichen Propargylfunktionen des Chromens kleiner als 11 % ist, wenn der aromatische Propargylether der allgemeinen Formel (I) Propargylresorcin ist, der prozentuale Molanteil an restlichen Propargylfunktionen des Chromens kleiner als 39 % ist, wenn der aromatische Propargylether der allgemeinen Formel (I) gekoppeltes Propargyleugenol ist, und der prozentuale Molanteil an restlichen Propargylfunktionen des Chromens kleiner als 35 % ist, wenn der aromatische Propargylether der allgemeinen Formel (I) gekoppeltes Propargylisoeugenol ist.

4. Verfahren nach einem der Ansprüche 1 bis 3, **dadurch gekennzeichnet, dass** es zwischen 1 und 1000 Stunden, vorteilhafterweise zwischen 2 und 50 Stunden dauert.

5. Verfahren nach einem der Ansprüche 1 bis 4, **dadurch gekennzeichnet, dass** der Katalysator ausgewählt ist aus CuCI und CuCl₂, vorteilhafterweise handelt es sich um CuCI.

6. Verfahren nach einem der Ansprüche 2 bis 5, **dadurch gekennzeichnet, dass** der aromatische Propargylether der allgemeinen Formel (I) Propargylresorcin ist und dass das erhaltene Chromen die folgende Formel C und/oder D aufweist, vorteilhafterweise eine Mischung aus den Formeln C und D ist. 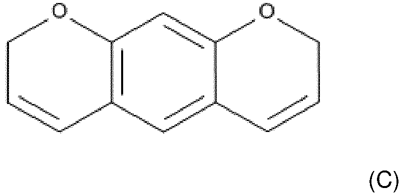 und

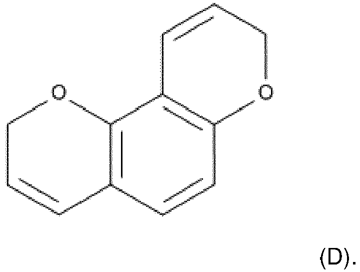

7. Verfahren nach einem der Ansprüche 1 bis 6, **dadurch gekennzeichnet, dass** der Katalysatorgehalt in dem Reaktionsmedium zwischen 10 und 10.000 ppm, vorteilhafterweise zwischen 500 und 1.500 ppm liegt.

8. Verfahren zur Herstellung eines Materials aus durch Wärme ausgehärtetem Harz, umfassend die folgenden aufeinanderfolgenden Schritte:
- a) Durchführung des Verfahrens nach einem der Ansprüche 1 bis 7,
- b) Polymerisation des in Schritt a) erhaltenen Reaktionsproduktes, um das Material aus durch Wärme ausgehärtetem Harz zu erhalten,
- c) Gewinnung des Materials aus durch Wärme ausgehärtetem Harz, das in Schritt b) erhalten wurde.

9. Verfahren nach Anspruch 8, **dadurch gekennzeichnet, dass** die Polymerisationsenthalpie von Schritt b) weniger als 500 J/g beträgt.

10. Verfahren nach einem der Ansprüche 8 oder 9, **dadurch gekennzeichnet, dass** der Koksgehalt des in Schritt c) erhaltenen durch Wärme ausgehärteten Harzes größer als 50 % ist.

## Claims

1. A process for producing chromenes intended for the preparation of thermosetting resins, comprising the step of converting an aromatic propargyl ether of general formula (I) below: 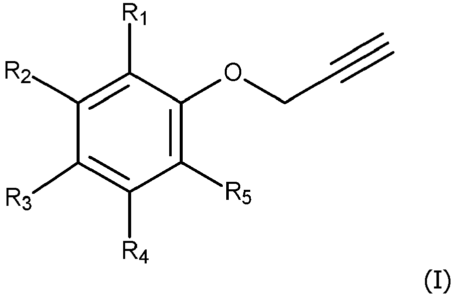 wherein:
**R₁ and R₅** represent, independently of one another, a hydrogen atom, or
a C₂-C₆ alkene, C₂-C₆ alkyne, O-(C₁-C₆)alkyl, O-(C₂-C₆)alkene or O-(C₂-C₆)alkyne group, on condition that at least one of **R₁ and R₅** represents a hydrogen atom;
**R₂ and R₄** represent, independently of one another, a hydrogen atom, a C₂-C₆ alkene group, a C₂-C₆ alkyne group such as a propargyl, an O-(C₁-C₆)alkyl group, an O-(C₂-C₆)alkene group or an O-(C₂-C₆)alkyne group, such as an O-propargyl;
**and R₃** represents a hydrogen atom, an O-(C₁-C₆)alkyl group or a C₂-C₆ alkene group, the alkene group being optionally substituted with a group of general formula (II) below: 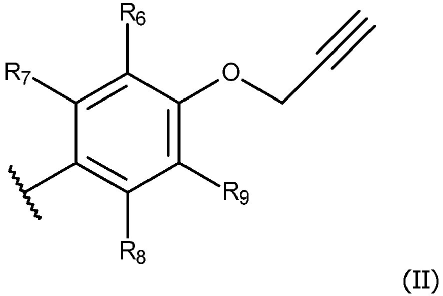
wherein:
**R₆ and R₉** represent, independently of one another, a hydrogen atom, or a C₂-C₆ alkene, C₂-C₆ alkyne, O-(C₁-C₆)alkyl, O-(C₂-C₆)alkene or O-(C₂-C₆)alkyne group, on condition that at least one of **R₆ and R₉** represents a hydrogen atom;
and **R₇ and R₈** represent, independently of one another, a hydrogen atom, a C₂-C₆ alkene group, a C₂-C₆ alkyne group such as a propargyl, an O-(C₁-C₆)alkyl group, an O-(C₂-C₆)alkene group or an O-(C₂-C₆)alkyne group such as an O-propargyl;
on condition that at least one of **R₁, R₂, R₃, R₄ and R₅** does not represent a hydrogen atom or an O-(C₁-C₆)alkyl group;
and the cis/trans isomers thereof and the optical isomers thereof, and racemic mixtures thereof
into a chromene by homogeneous catalysis with copper salts in anisole at a temperature of between 100 and 170°C, advantageously between 120 and 160°C.

2. The process as claimed in claim 1, wherein the aromatic propargyl ether of general formula (I) is chosen from the group consisting of propargylated resorcinol, propargylated eugenol, propargylated coupled eugenol, propargylated coupled isoeugenol, propargylated isoeugenol and mixtures thereof and the cis/trans isomers thereof and the optical isomers thereof and racemic mixtures thereof, it is advantageously propargylated resorcinol.

3. The process as claimed in claim 2, wherein the molar percentage of residual propargyl functions of the chromene is less than 11% when the aromatic propargyl ether of general formula (I) is propargylated resorcinol, the molar percentage of residual propargyl functions of the chromene is less than 39% when the aromatic propargyl ether of general formula (I) is propargylated coupled eugenol and the molar percentage of residual propargyl functions of the chromene is less than 35% when the aromatic propargyl ether of general formula (I) is propargylated coupled isoeugenol.

4. The process as claimed in any one of claims 1 to 3, which lasts between 1 and 1000 hours, advantageously between 2 and 50 hours.

5. The process as claimed in any one of claims 1 to 4, wherein the catalyst is chosen from CuCl and CuCl₂, it is advantageously CuCl.

6. The process as claimed in any one of claims 2 to 5, wherein the aromatic propargyl ether of general formula (I) is propargylated resorcinol and wherein the chromene obtained has the formula C and/or D below; it is advantageously a mixture of the formulae C and D 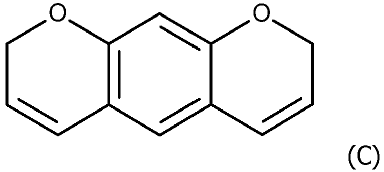 and

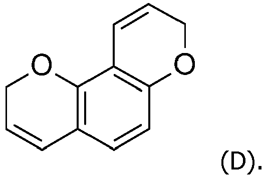

7. The process as claimed in any one of claims 1 to 6, wherein the catalyst content in the reaction medium is between 10 and 10 000 ppm, advantageously between 500 and 1500 ppm.

8. A process for preparing a material made of thermoset resin, comprising the following successive steps:
- a) carrying out the process as claimed in any one of claims 1 to 7;
- b) polymerizing the reaction product obtained in step a) so as to obtain the material made of thermoset resin;
- c) recovering the material made of thermoset resin obtained in step b).

9. The process as claimed in claim 8, wherein the enthalpy of polymerization of step b) is less than 500 J/g.

10. The process as claimed in either one of claims 8 and 9, wherein the coke content of the thermoset resin obtained in step c) is greater than 50%.
